# EUROPEAN PATENT APPLICATION

(11) **EP 4 374 845 A1**
(43) Date of publication of application: **29.05.2024**
(21) Application number: 22846227.1
(22) Date of filing: 20.07.2022
(51) Int. Cl.: A61K 8/66, A61K 8/99, A61Q 11/00, A61K 38/44, A61K 35/742, A61P 1/02, A23L 33/17, A23L 33/135, A23K 20/147, A23K 10/16

(54) **COMPOSITION COMPRISING SUPEROXIDE DISMUTASE AND/OR BACILLUS STRAIN SPORES, AND USE THEREOF FOR IMPROVING ORAL HEALTH**

(30) Priority: 21.07.2021 KR 20210095682
(71) Applicant: Genofocus, Inc., Daejeon 34014 (KR); BiomLogic, Inc., Seoul 03923 (KR); Vexpert Co., Ltd., Yongin-si, Gyeonggi-do 16942 (KR)
(72) Inventor: PAN, Jae Gu, Seoul 03923 (KR); KIM, Eui Joong, Daejeon 34014 (KR); KIM, Jeong Hyun, Daejeon 34014 (KR); CHANG, In Ik, Seoul 03923 (KR); PARK, Yung Chan, Yongin-si, Gyeonggi-do 16942 (KR)
(74) Representative: Dehns
(86) International application number: PCT/KR2022/010623
(87) International publication number: WO 2023/003357

(57) **Abstract**

The present invention relates to an oral composition comprising the superoxide dismutase (SOD) enzyme and/or Bacillus spp. strain spores, and a use thereof for improving oral health or managing oral hygiene. The composition may be useful for preventing or treating oral diseases such as oral pain, halitosis, tartar, plaque, gingivitis, and periodontitis, without causing adverse effects in the oral cavity.

## Description

### Technical Field

The present disclosure relates to a composition comprising a superoxide dismutase enzyme and/or a *Bacillus sp.* strain spore, and a use thereof for promoting oral health, managing oral hygiene, or preventing or treating an oral disease.

### Background Art

Most oral diseases begin with poor oral hygiene, leading to calculus, cavity, gingivitis, or the like, and may be accompanied by severe bad breath. The main causes of bad breath, also called halitosis, include a periodontal disease such as gingivitis and periodontitis, odor-causing bacteria on the tongue, certain foods and alcoholic beverages, smoking, and the like. In a case where food in the mouth is not properly removed, soft deposits called plaque form on the tooth surface, and the plaque turns into calculus over time. Calculus is caused by calcification of minerals from saliva and gingival crevicular fluid and is firmly adhered to the tooth surface. Calculus cannot be removed by toothbrushing and can only be removed by scaling. In a case where calculus is not removed, the calculus easily causes inflammation called gingivitis in the gums. As gingivitis worsens, inflammation occurs not only in the gums but also in the ligaments or the alveolar bone. Such inflammation is periodontitis, which also causes bad breath. Periodontitis not only causes extreme pain, but also can lead to bacterial infection even in the heart or kidneys. Thus, periodontitis requires special attention.

Substances, which inhibit growth of oral pathogens that live in plaque and cause various oral diseases, include antibacterial agents including antibiotics. However, antibiotics have the disadvantage of having to be used as a therapeutic agent for a short period of time as it is difficult to use the antibiotics for a long period of time because they can cause systemic side effects on the human body and emergence of resistant bacteria in the oral cavity. In addition, antibacterial agents used in oral gargles include sanguinarine, LISTERINE^{®}, chlorhexidine, and the like. Among these, sanguinarine has the disadvantage of not only being unclear about its effectiveness against bacteria in the oral cavity, but also being expensive; and LISTERINE^{®}, which contains alcohol as its main ingredient, has a slight bacteriostatic effect and has the disadvantage of only having a temporary effect in the oral cavity and having a harmful effect on tissues when used for a long period of time.

An effective way to remove calculus is scaling. However, scaling may cause gums to become swollen or sensitive to cold or hot foods, and improper scaling may cause gum damage.

Therefore, there is a need for materials and methods that can be used for oral hygiene management and oral health promotion, as well as for preventing or treating an oral disease, without causing adverse side effects on teeth or gums.

### Disclosure of Invention

### Technical Problem

The object of the present disclosure is to solve the above-mentioned problems of the prior art.

Another object of the present disclosure is to promote oral health or manage oral hygiene using a superoxide dismutase and/or a *Bacillus sp.* strain spore.

Yet another object of the present disclosure is to prevent or treat an oral disease using a superoxide dismutase and/or a *Bacillus sp.* strain spore.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an oral composition, comprising at least one selected from the group consisting of a superoxide dismutase (SOD) and a *Bacillus sp.* strain spore.

According to another aspect of the present disclosure, there is provided an oral product, comprising the composition.

According to yet another aspect of the present disclosure, there is provided a pharmaceutical composition for preventing or treating an oral disease, comprising the composition.

According to still yet another aspect of the present disclosure, there is provided a veterinary composition, comprising the composition.

According to still yet another aspect of the present disclosure, there is provided a food composition, comprising the composition.

According to still yet another aspect of the present disclosure, there is a feed composition, comprising the composition.

According to still yet another aspect of the present disclosure, there is provided a method for promoting oral hygiene, or preventing or treating an oral disease, comprising administering to an individual at least one selected from the group consisting of a superoxide dismutase (SOD) and a *Bacillus sp.* strain spore, or administering to an individual the composition.

### Advantageous Effects of Invention

The composition according to the present disclosure can be effectively used to prevent or treat an oral disease such as halitosis, calculus, plaque, dental caries, gingivitis, or periodontitis, as well as to reduce pain caused by such an oral disease, without causing side effects.

### Brief Description of Drawings

FIG. 1 illustrates criteria for converting calculus index into a score.
FIG. 2 illustrates criteria for converting gingivitis index into a score.
FIG. 3 illustrates a graph showing results of halitosis index observed after administration of a test material, according to Experimental Example 1.4.
FIG. 4 illustrates a graph showing results of calculus index observed after administration of a test material, according to Experimental Example 1.4. * indicates *P* < 0.05 relative to week 0.
FIG. 5 illustrates a graph showing results of plaque index observed after administration of a test material, according to Experimental Example 1.4.
FIG. 6 illustrates a graph showing results of gingivitis index observed after administration of a test material, according to Experimental Example 1.4. * * indicates *P* < 0.01 relative to week 0.
FIG. 7 illustrates a graph showing results of palpable pain index observed after administration of a test material, according to Experimental Example 2.4. * indicates *P* < 0.05 relative to week 0.
FIG. 8 illustrates a graph showing results of halitosis index observed after administration of a test material, according to Experimental Example 2.4.
FIG. 9 illustrates a graph showing results of plaque index observed after administration of a test material, according to Experimental Example 2.4. * indicates *P* < 0.05 relative to week 0, and ** indicates *P* < 0.01 relative to week 0.
FIG. 10 illustrates a graph showing results of calculus index observed after administration of a test material, according to Experimental Example 2.4. * indicates *P* < 0.05 relative to week 0.
FIG. 11 illustrates a graph showing results of gingivitis index observed after administration of a test material, according to Experimental Example 2.4. ** indicates *P* < 0.01 relative to week 0.
FIG. 12 illustrates an overall procedure for producing a recombinant production strain (BSBA310) that expresses SodA2.
FIG. 13 illustrates an expression vector for overexpression of *sodA2* gene. Here, rrnB T1T2 represents a transcription terminator; rep(pBR322) represents a pBR322-derived replicon that operates in E. coli.; rep(pUB 110) represents a pUB 110-derived replicon that operates in B. subtilis; KanR represents a kanamycin resistance gene (aminoglycoside O-nucleotidyltransferase); and BJ27 promoter represents a strong promoter for B. subtilis.

### Best Mode for Carrying out Invention

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

### Definition

As used herein, the term "individual" is used interchangeably with "patient," and may be any mammal in need of oral hygiene or prevention or treatment of an oral disease, such as primate (for example, human, monkey, and chimpanzee), companion animal (for example, dog and cat), domestic animal (for example, cow, pig, horse, sheep, and goat), or laboratory animal (for example, rat, mouse, and guinea pig).

The term "treatment" generally refers to obtaining a desired pharmacological and/or physiological effect. Such an effect has a therapeutic effect in that it partially or completely cures a disease and/or other unwanted or undesirable conditions (for example, halitosis, calculus, plaque, dental caries, gingivitis, periodontitis, and pain). Desirable therapeutic effects include, but are not limited to, prevention of occurrence or recurrence of a disease, amelioration of symptoms, reduction of any direct or indirect pathological consequences of a disease, prevention of metastasis, reduction of disease progression rate, improvement or alleviation of disease state, and remission or improved prognosis. Preferably, the "treatment" may mean medical intervention for a disease or disorder that has already occurred.

The term "prevention" refers to obtaining a desired prophylactic pharmacological and/or physiological effect in terms of partially or completely preventing a disease or its symptoms.

The term "administration" refers to providing an active ingredient to an individual to achieve a prophylactic or therapeutic purpose.

### Oral composition

The present disclosure is based, in part, on the discovery that in a case of applying, to the oral cavity of an individual, a superoxide dismutase (SOD) or a *Bacillus sp*. strain spore alone or a combination of a superoxide dismutase (SOD) and a *Bacillus sp.* strain spore, promotion of oral hygiene or prevention or treatment of an oral diseases is effectively achieved. Accordingly, according to an aspect of the present disclosure, there is provided an oral composition, comprising a superoxide dismutase (SOD), a *Bacillus sp.* strain spore, or an SOD and a *Bacillus sp.* strain spore. Preferably, the composition comprises a superoxide dismutase enzyme and a *Bacillus sp.* strain spore.

A superoxide dismutase (SOD) is an enzyme that alternately catalyzes dismutation of superoxide (O₂^{•-}) radical into either ordinary molecular oxygen (O₂) or hydrogen peroxide (H₂O₂). SOD plays a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, or chloroplasts, mitochondria, and cytosol of eukaryotic cells. In the present disclosure, the term "SOD" may be used interchangeably with a (poly)peptide having superoxide dismutase activity. In addition, the SOD may include a polypeptide having superoxide dismutase activity or a fragment thereof, or a fusion containing the same.

In some embodiments, the SOD may bind manganese (Mn-SOD). Preferably, the SOD may be deamidated Mn-SOD. In addition, the SOD may comprise or consist of the amino acid sequence represented by SEQ ID NO: 2 (SodA). Preferably, the SOD may be one in which amino acid residues 74 and 137 are substituted with Asp based on SEQ ID NO: 2. More preferably, the SOD may comprise or consist of the amino acid sequence represented by SEQ ID NO: 4 (SodA2).

The SOD or polypeptide having SOD activity of the present disclosure is interpreted to include amino acid sequences showing substantial identity to the above-mentioned amino acid sequences. The substantial identity means that amino acid sequences show sequence identity of 80% or higher, preferably 90% or higher, more preferably 95% or higher, and most preferably 98% or higher in a case where the aligned sequences are analyzed using an algorithm commonly used in the art.

In some embodiments, the SOD may be a modified or engineered polypeptide having SOD enzymatic activity, the polypeptide comprising at least one mutation, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art including HIS tag, HA tag, and myc tag, GFC, and/or Fc domain of an antibody) for purification, detection, in vivo delivery, or increased stability.

In some embodiments, the SOD of the present disclosure may be derived from a variety of sources, including natural, mutant, or recombinant microorganisms. For example, the SOD may be derived from bacteria. Preferably, the SOD may be derived from bacteria that are generally regarded as safe (GRAS) for use in drugs or foods, such as *Bacillus sp.* strains or variants thereof. More preferably, the SOD may be obtained from *Bacillus amyloliquefaciens* strains (for example, GF423 or GF424 strain) or culture supernatants thereof. The GF423 and GF424 strains were deposited with the Korea Research Institute of Bioscience and Biotechnology on March 6, 2017 and March 13, 2017, respectively (under accession numbers KCTC 13222 BP and KCTC 13227 BP, respectively). The *Bacillus amyloliquefaciens* GF424 strain is a strain obtained by subjecting the *Bacillus amyloliquefaciens* GF423 strain to mutagenesis using UV irradiation to improve expression of *sodA* gene. The SOD enzyme (SodA) derived from the *Bacillus amyloliquefaciens* GF423 or GF424 strain is Mn-SOD and has the amino acid sequence of SEQ ID NO: 2 (the nucleotide sequence of which is represented by SEQ ID NO: 1). In addition, the SOD may be a recombinant polypeptide. For example, the SOD may be a deamidated SOD (SodA2), in which amino acid residues 74 and 137 are substituted with Asp based on SEQ ID NO: 2, having the amino acid sequence of SEQ ID NO: 4 (the nucleotide sequence of which is represented by SEQ ID NO: 3). The sequences of SEQ ID NOS: 1 to 4 are as shown in Table 1.

**[Table 1]**

| Description | Sequence | SEQ ID NO |
|---|---|---|
| Nucleotide sequence of SodA | ATGGCTTACAAACTTCCAGAATTGCCTTACGCTTAT GATGCTTTAGAACCTCATATCGATAAGGAAACGATG ACGATTCACCATACGAAGCACCATAACACATACGTG ACAAACCTCAACAAAGCGATCGAAGGATCTGCGCT TGCAGAGAAATCTGTAGATGAGCTTGTTGCTGATTT GAACGCAGTGCCGGAGGACATCCGCACGGCAGTCC GCAACAATGGCGGCGGACATGCAAACCACTCTTTA TTCTGGACTCTTTTATCTCCGAACGGCGGAGGCGAA CCGACTGGTGAGCTTGCTGAAGAGATCAAAAGCAC GTTCGGAAGCTTCGATCAATTTAAAGAAAAATTCGC CGCAGCAGCTGCAGGCCGTTTCGGTTCAGGCTGGG CTTGGCTCGTTGTAAACAACGGCAAACTTGAAATTA CAAGCACGCCAAACCAAGATTCACCGCTTTCAGAA GGTAAAACACCTGTTCTCGGTCTTGATGTTTGGGAG CATGCGTACTACCTGAACTACCAAAACCGCCGTCCT GATTACATTTCAGCTTTCTGGAATGTTGTGAACTGG GATGAAGTTGCCCGTCTTTACAGCGAAGCAAAATA A | SEQ ID NO: 1 |
| Amino acid sequence of SodA | MAYKLPELPYAYDALEPHIDKETMTIHHTKHHNTYV TNLNKAIEGSALAEKS VDELVADLNAVPEDIRTAVRN NGGGHANHSLFWTLLSPNGGGEPTGELAEEIKSTFGS FDQFKEKFAAAAAGRFGSGWAWLVVNNGKLEITSTP NQDSPLSEGKTPVLGLDVWEHAYYLNYQNRRPDYIS AFWNVVNWDEVARLYSEAK | SEQ ID NO: 2 |
| Nucleotide sequence of SodA2 | ATGGCTTACAAACTTCCAGAATTGCCTTACGCTTAT GATGCTTTAGAACCTCATATCGATAAGGAAACGATG ACGATTCACCATACGAAGCACCATAACACATACGTG | SEQ ID NO: 3 |
| | ACAAACCTCAACAAAGCGATCGAAGGATCTGCGCT TGCAGAGAAATCTGTAGATGAGCTTGTTGCTGATTT GAACGCAGTGCCGGAGGACATCCGCACGGCAGTCC GCAACGATGGCGGCGGACATGCAAACCACTCTTTA TTCTGGACTCTTTTATCTCCGAACGGCGGAGGCGAA CCGACTGGTGAGCTTGCTGAAGAGATCAAAAGCAC GTTCGGAAGCTTCGATCAATTTAAAGAAAAATTCGC CGCAGCAGCTGCAGGCCGTTTCGGTTCAGGCTGGG CTTGGCTCGTTGTAAACGACGGCAAACTTGAAATTA CAAGCACGCCAAACCAAGATTCACCGCTTTCAGAA GGTAAAACACCTGTTCTCGGTCTTGATGTTTGGGAG CATGCGTACTACCTGAACTACCAAAACCGCCGTCCT GATTACATTTCAGCTTTCTGGAATGTTGTGAACTGG GATGAAGTTGCCCGTCTTTACAGCGAAGCAAAATA A | |
| Amino acid sequence of SodA2 | MAYKLPELPYAYDALEPHIDKETMTIHHTKHHNTYV TNLNKAIEGSALAEKS VDELVADLNAVPEDIRTAVRN DGGGHANHSLFWTLLSPNGGGEPTGELAEEIKSTFGS FDQFKEKFAAAAAGRFGSGWAWLVVNDGKLEITSTP NQDSPLSEGKTPVLGLDVWEHAYYLNYQNRRPDYIS AFWNVVNWDEVARLYSEAK | SEQ ID NO: 4 |

In Addition, the SOD may be derived from other recombinant strains (for example, *Bacillus subtilis sp.* strain) using the SOD-expressing gene of the *Bacillus amyloliquefaciens* strain. The recombinant strain may be produced by a recombinant technique using a conventional protein-producing strain known in the art. For example, the *Bacillus subtilis* strain (which is a parent strain of the recombinant strain) may be KCTC 3135, and the KCTC 3135 strain may be obtained from the Korean Collection for Type Cultures (KCTC), Biological Resource Center, Korea Research Institute of Bioscience and Biotechnology. In addition, in the recombinant strain, one or more of the genes shown in Table 2 may be removed to facilitate subsequent processing.

**[Table 2]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX prophage gene |

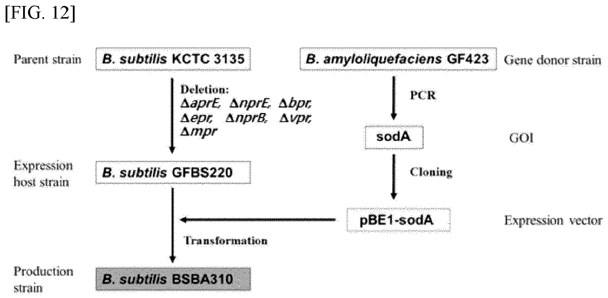

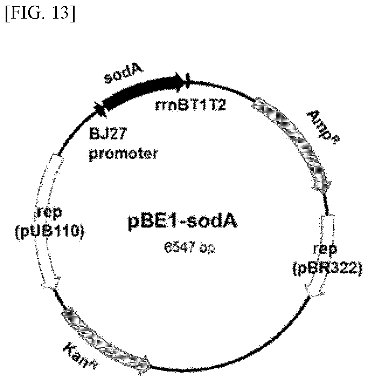

For example, the recombinant strain may be produced through the process shown in FIG. 12. In addition, the recombinant strain may comprise the expression vector shown in FIG. 13. In the drawing, sodA and sodA2 represent SOD-coding genes.

From the viewpoint that the SOD derived from the above-mentioned strain is an enzyme secreted outside of a cell, in a case where the SOD is produced using the strain, it is possible to mass-produce an SOD that is safe for individuals without going through an expensive purification process (for example, column purification), which enables efficient production.

In some embodiments, the SOD of the present disclosure may be obtained by culturing the natural, mutant, or recombinant microorganism in various culture media. For example, the SOD to be included in an oral composition may be isolated from a culture supernatant of the *Bacillus amyloliquefaciens* GF423 or GF424 strain. Specifically, the *Bacillus amyloliquefaciens* strain may be first cultured in various types of media to obtain a culture. For example, the strain is grown at about 25°C to about 42°C for about 1 to about 4 days using a complex medium (pH 6.0 to 7.0). Other suitable media for culturing the *Bacillus amyloliquefaciens* strain include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. In a preferred embodiment, LB medium, ISP medium, BHI medium, SDA medium, or NB medium may be used. In addition, the SOD may be obtained from other natural, mutant, or recombinant hosts using information provided in databases such as PubMed or BRENDA (www.brenda-enzymes.org).

In some embodiments, the SOD of the present disclosure may be isolated or purified from a culture of a natural, mutant, or recombinant strain. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein, in which the protein is separated from cellular components of the cell from which the protein is isolated or recombinantly produced. In some embodiments, the phrase "substantially free of cellular material" includes preparations of a protein, having less than about 30%, preferably less than about 20%, more preferably less than about 10%, and most preferably less than about 5% by dry weight of unwanted protein.

The SOD may be preferably purified by the following purification method, but the method is not limited thereto. For example, the culture previously obtained by culturing the *Bacillus amyloliquefaciens* strain is centrifuged to collect a culture supernatant. The supernatant fraction is pretreated by solid-phase extraction, and then isolated and purified by chromatography. Various modes of chromatography may be used to purify an SOD. Preferably, hydrophobic interaction chromatography is used.

In some embodiments, the SOD may be included as a strain lysate, strain culture, strain culture concentrate, or strain culture extract, or a dried form thereof. Here, the "strain lysate" refers to a product obtained by culturing the strain and disrupting it mechanically or chemically, and may include any product obtained therefrom through additional processes such as extraction, dilution, concentration, and purification. The "strain culture" may refer to a culture itself obtained by culturing the strain or a supernatant thereof. The "strain culture concentrate" refers to a product that is separated purely from the strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated liquid of the extract, a dried product obtained by drying the extract, or a crude or purified product thereof, or a fraction obtained by fractionating the same. The dried form may include a lyophilized form.

In some embodiments, the SOD may comprise a cellular material from the cell or tissue source from which it is derived, such as extracellular vesicle. In this case, the cellular material containing an SOD may be obtained by culturing various sources, including natural, mutant, or recombinant hosts, using conventional techniques known in the art as described above, and isolating the cellular material from the culture using methods such as filtration and concentration.

Meanwhile, the *Bacillus sp.* strain spores may be obtained by culturing a *Bacillus sp.* strain in a suitable medium, inducing sporulation, and then isolating the resulting spores. In an embodiment, the *Bacillus sp.* strain spores may be obtained from GRAS bacteria that are generally regarded as safe for use in drugs or foods. *Bacillus sp.* strain spores are known to be resistant to proteases and low pH (Cutting SM. Bacillus probiotics. Food Microbiol. 2011;28:214-220. doi: 10.1016/j.fm.2010.03.007; and Wang Y, et al., In vitro assessment of probiotic properties of Bacillus isolated from naturally fermented congee from inner Mongolia of China. World J. Microb. Biot. 2010;26:1369-1377. doi: 10. 1007/s 11274-0 10-0309-7). In addition, *Bacillus sp.* strain spores are GRAS probiotics approved in several countries. Specifically, the *Bacillus sp.* strain spores may be derived from *Bacillus amyloliquefaciens* strains (for example, GF423 or GF424 strain). Sporulation may be induced using conventional techniques known in the art. For example, the *Bacillus sp.* strain spores are spores of a strain obtained by pre-culturing the strain and then inducing sporulation in media such as DSM, NB, SYP, and LB2 during main culture of the precultured strain, and may be obtained by culturing various sources, which include natural, mutant, or recombinant hosts, removing vegetative cells therefrom, and then isolating the spores through filtration, concentration, or centrifugation.

In some embodiments, the oral composition may comprise an SOD and a *Bacillus sp.* strain spore. According to an embodiment of the present disclosure, it was identified that in a case where a mixture of an SOD and a *Bacillus sp.* strain spore is applied to the oral cavity, the mixture exhibited excellent effects in preventing, ameliorating, or treating halitosis, calculus, plaque, dental caries, or a periodontal disease (for example, gingivitis or periodontitis), and thus was effective in promoting oral health, managing oral hygiene, or preventing or treating an oral disease. In addition, it was identified that the mixture was effective in reducing pain caused by an oral disease.

In some embodiments, the SOD and the *Bacillus sp.* strain spores may be mixed in an appropriate ratio to exert such an effect. For example, the SOD and the *Bacillus sp.* strain spores may be mixed in a ratio such that the SOD has a titer of 5,000 to 20,000 U per g dry weight and the spores are present in 0.1 × 10^10 to 1 × 10^11 cfu per g dry weight.

In some embodiments, the composition may be used to promote oral health or manage oral hygiene.

In some embodiments, the composition may be used for prevention or treatment of an oral disease.

In some embodiments, the composition may be used to prevent, ameliorate, or treat at least one disease selected from halitosis, calculus, plaque, dental caries, and a periodontal disease.

In some embodiments, the composition may be used to reduce oral pain.

In other embodiments, the composition may have various forms for oral application known in the art, including a liquid form, a solid form, a gel form, a powder form, a paste form, or a form impregnated in or applied to a carrier, and the like.

### Oral product

According to another aspect of the present disclosure, there is provided an oral product, comprising the oral composition. In other words, the oral product comprises at least one selected from the group consisting of the SOD and the *Bacillus sp.* strain spore as described above.

The product may be at least one selected from, but is not limited to, the group consisting of toothpaste, mouthwash, oral gargle, gum, candy, oral spray, oral gel, oral ointment, oral patch, and oral rinse.

The oral product of the present disclosure may comprise additional ingredients known in the art necessary for formulation thereof, in addition to comprising as an active ingredient(s) the SOD and/or the *Bacillus sp.* strain spore. For example, in a case where the oral product is toothpaste, it may further comprise an abrasive, a moisturizer, a foaming agent, a sweetener, a whitening agent or a flavoring agent, and the like. In addition, in a case where the oral product is an oral gel, it may further comprise a thickener, such as medium chain fatty acid, for viscosity or adhesion of the gel, and the like. In addition, in a case where the oral product is an oral rinse, it may further comprise a carrier such as non-toxic alcohol.

### Pharmaceutical or veterinary composition

According to yet another aspect of the present disclosure, there is provided a pharmaceutical or veterinary composition, comprising a composition that comprises at least one selected from the group consisting of the superoxide dismutase (SOD) enzyme and *Bacillus sp.* strain spore as described above. The pharmaceutical composition of the present disclosure may be used interchangeably with the term "veterinary composition" in a case of being applied to an animal other than a human.

In some embodiments, the composition may be used for prevention or treatment of an oral disease. Specifically, the oral disease may be at least one disease selected from, but is not limited to, halitosis, calculus, plaque, dental caries, a periodontal disease, and chronic stomatitis.

In some embodiments, the composition may be used for prevention or treatment of oral pain. The oral pain may be caused various oral diseases or undesirable conditions. For example, the oral pain may be caused by calculus, plaque, dental caries, a periodontal disease, or chronic stomatitis.

The pharmaceutical or veterinary composition of the present disclosure may further comprise at least one selected from the group consisting of pharmaceutically acceptable carriers, excipients, and diluents. The pharmaceutically acceptable carrier, excipient, and/or diluent may be those commonly used in the art. The carrier, excipient, or diluent may include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, propyl hydroxybenzoate, talc, magnesium stearate, silicon dioxide, and mineral oil, but are not limited thereto.

In a case of performing preparation, the preparation may be achieved by using an additive such as a filler, an extender, a binder, a wetting agent, a disintegrant, or a surfactant. The additive for the preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

The pharmaceutical or veterinary composition of the present disclosure may be administered orally or parenterally depending on the intended method. In a case of being administered parenterally, it is preferable to select an injection method such as nasal spray, external application to the skin or intraperitoneal injection, intrarectal injection, subcutaneous injection, intravenous injection, intramuscular injection, or intrathoracic injection. In addition, the pharmaceutical or veterinary composition of the present disclosure may be preferably applied through an intraoral injection method such as oral spray, oral application, or intraoral injection.

Here, solid preparations for oral administration include tablets, pills, powders, granules, capsules, and the like, and these solid preparations may be prepared by mixing the composition with at least one excipient such as starch, calcium carbonate, sucrose, lactose, and gelatin. Further, in addition to simple excipients, a lubricant such as magnesium stearate and talc may also be used. Examples of liquid preparations for oral administration include suspensions, solutions, emulsions, and syrups. In addition to commonly used simple diluents such as water and liquid paraffin, various types of excipients, such as a wetting agent, a sweetener, a fragrance, and a preservative, may be used. For oral administration, the SOD may be coated with a coating agent such as shellac, ethyl cellulose, hydroxypropyl methylcellulose, hydroxypropyl methylcellulose phthalate, zein, Eudragit, and combinations thereof; however, the coating agent is not limited thereto.

Forms for parenteral administration include toothpaste, mouthwash, formulation for topical administration (for example, gel, cream, ointment, dressing solution, spray, and other agents for application), and the like. An example of the formulation for topical administration may be one in which the active ingredient of the present disclosure is impregnated into a carrier such as a gauze made of natural fiber or synthetic fiber. The gel, cream, or ointment may be suitable for direct application to or around an affected or decayed area, including teeth and gums. The spray may be produced by a typical spray-producing method, and may be used to prevent or treat an oral disease in such a manner that it is filled and packaged in a compression container or other spray container to be often sprayed and applied to an oral disease area. The dressing solution may be produced by a typical dressing solution-producing method, and may be used to prevent or treat an oral disease in such a manner that it dresses an oral disease area or other bacterial infection area.

The pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

### Food or feed composition

According to still yet another aspect of the present disclosure, there is provided a food composition, comprising a composition that comprises at least one selected from the group consisting of the superoxide dismutase (SOD) enzyme and *Bacillus sp.* strain spore as described above. Such a food composition includes a medical or nutraceutical food composition.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto, and does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of promoting oral hygiene or preventing or ameliorating an oral disease. Specific examples of the carrier, excipient, diluent, and additive are well known in the art, and those skilled in the art are capable of combining appropriate ingredients depending on the formulation to manufacture the food.

An amount of the superoxide dismutase enzyme and/or *Bacillus sp.* strain spores according to the present disclosure as an active ingredient(s) in the above-described formulation may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on the patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

According to still yet another aspect of the present disclosure, there is provided a feed composition, comprising a composition that comprises at least one selected from the group consisting of the superoxide dismutase (SOD) enzyme and *Bacillus sp.* strain spore as described above. In addition, the feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient that is added to feed (calcium carbonate, wheat shorts, zeolite, corn meal, rice bran, or the like).

### Method for promoting oral hygiene, or preventing or treating oral disease

According to still yet another aspect of the present disclosure, there is provided a method for promoting oral hygiene, comprising administering to an individual at least one selected from the group consisting of the superoxide dismutase (SOD) enzyme and *Bacillus sp.* strain spore as described above. In addition, there is provided a method for preventing or treating an oral disease, comprising administering to an individual at least one selected from the group consisting of the superoxide dismutase (SOD) enzyme and *Bacillus sp.* strain spore as described above.

In some embodiments, the method may comprise administering to the individual a combination of the SOD and the *Bacillus sp.* strain spore.

In some embodiments, the oral disease may be at least one selected from the group consisting of oral pain, halitosis, plaque, calculus, dental caries, and periodontal disease.

In an embodiment, in a case of being administered in combination, the SOD and the *Bacillus sp.* strain spore may be administered simultaneously as a single composition or as separate compositions comprising each of them. In addition, the SOD and the *Bacillus sp.* strain spore may be administered sequentially or in reverse order as separate compositions comprising each of them. In a case of being administered simultaneously or separately, the SOD and the *Bacillus sp.* strain spores may be administered through the same route or different routes, depending on the dosage form. The composition, dosage form, route of administration, and the like are as described above for the composition.

Hereinafter, the present disclosure will be described in detail by way of examples to help understanding. However, the following examples are only for illustrating the present disclosure, and the scope of the present disclosure is not limited thereto.

### Examples

### Example 1. Isolation and purification of superoxide dismutase from Bacillus amyloliquefaciens strain

The *Bacillus amyloliquefaciens* strains used in the present example are *Bacillus amyloliquefaciens* GF423 strain and *Bacillus amyloliquefaciens* GF424 strain, and these strains were deposited with the Korea Research Institute of Bioscience and Biotechnology under accession numbers KCTC 13222 BP and KCTC 13227BP on March 6, 2017 and March 13, 2017, respectively.

The *Bacillus amyloliquefaciens* GF424 strain is a strain obtained by subjecting the *Bacillus amyloliquefaciens* GF423 strain to mutagenesis using UV irradiation to improve expression *of sodA* gene.

### Example 1.1. Culture of Bacillus amyloliquefaciens GF423 or GF424 strain

For culture of *Bacillus amyloliquefaciens* GF423 or GF424 strain, a single colony formed on LB agar medium (Luria-Bertani (LB) agar; 10 g/L of tryptophan, 5 g/L of yeast extract, 10 g/L of NaCl, 15 g/L of agar) was inoculated into 30 ml of LB medium and cultured at 37°C for 12 hours. This seed culture was again inoculated into 3 L of LB medium containing 1 mM manganese sulfate (MnSO₄), and cultured at 37°C for 20 hours.

### Example 1.2. Isolation and purification of superoxide dismutase (SOD)

The cell culture obtained in Example 1.1 was centrifuged at 4°C and 3,578 x g for 20 minutes to collect the supernatant. Then, the supernatant was concentrated 10 times using ultrafiltration (hereinafter referred to as UF, MWCO 10,000). The concentrate was filtered through a sterilization filter and then lyophilized. Activity of an SOD was analyzed using an SOD assay kit (Cayman Chemical, Michigan, USA). One (1) unit of SOD activity is defined as an amount of enzyme that inhibits superoxide radicals by 50%. Activity of the dried SOD enzyme was 30 ± 6 U/mg.

### Example 2. Preparation of Bacillus amyloliquefaciens strain spores

### Example 2.1. Composition of medium

The medium used in the present example was SYP or DSM. The SYP medium contains 1.5% soy tone, 0.5% yeast extract, 0.5 % K₂HPO₄, 0.1% MnSO₄, 0.1% MgSO₄, 10 mM FeSO₄, 0.04% (NH₄)₂SO₄, 0.04% (NH₄)₂PO₄, 0.1% CaCl₂, and 2% glucose. The DSM medium contains 8 g/L of Bacto nutrient broth, 1 g/L of KCl, 0.25 g/L of MgSO₄, 0.16415 g/L of Ca(NO₃)₂, 0.9521 mg/L of MnCl₂, and 0.152 mg of FeSO₄. MnSO₄, MgSO₄, FeSO₄, (NH₄)₂SO₄, (NH₄)₂PO₄, and CaCl₂ were dissolved in ddH₂O before use and added.

### Example 2.2. Induction of sporulation

A single colony of the *Bacillus amyloliquefaciens* GF423 or GF424 strain was inoculated into 1 mL of LB contained in a 14 mL tube, and cultured at 37°C and 200 rpm for 12 hours. 1 mL of culture was transferred to 50 mL of LB medium in a 500 mL flask, and cultured at 37°C and 200 rpm for 12 hours. Then, 20 mL of culture medium was transferred to 1 L of SYP or DSM in a 2.5 L baffled flask. The inoculated culture was cultured at 37°C and 200 rpm for 24 to 120 hours.

### Example 2.3. Washing spores

After culture, lysozyme (0.5 g/L) was added to the culture broth and incubated at 37°C and 200 rpm for 1 hour to remove remaining vegetative cells. Centrifugation was performed at 6000 rpm for 10 minutes to collect crude spores. For purification, the collected crude spores were washed with water twice, washed with 0.02% SDS, washed again with water twice, and then suspended in a PBS solution. The spore suspension was kept at -20°C. The number of spores was determined by spreading the diluted spore solution on an LB agar plate and then counting colonies.

### Experimental Example 1. Animal tests: dogs

Tests using beagle dogs were performed to identify whether a mixture of the superoxide dismutase (SOD) purified in Example 1 and the *Bacillus amyloliquefaciens* strain spores prepared in Example 2 is effective in promoting oral health and managing oral hygiene. These tests were approved by the Institutional Animal Care and Use Committee of Chaon Co., Ltd. based on the Animal Protection Act (IACUC approval number: CE21115).

### Experimental Example 1.1. Test material

The test material used was prepared by mixing the superoxide dismutase (SOD) purified from *Bacillus amyloliquefaciens* GF424 in Example 1 and the *Bacillus amyloliquefaciens* GF423 strain spores prepared in Example 2 (2.5% mixture, 10,000 U/g of SOD).

Specifically, the mixture was obtained by dissolving medium-chain fatty acid, such as lauric acid, or the like, which is used to maintain viscosity/adhesion of an oral gel at room temperature, to achieve adjustment to a desired viscosity, performing mixing with a palatable ingredient such as betaine, oligosaccharide, and egg yolk, and then finally performing homogenous mixing with the SOD and 2.5% of the spores (SOD titer: 12,510 U/g, spores: 1.2 x 10^10 CFU/g). This mixture (SOD + spores) was placed in a tube in a form of an oral gel, which is gel-type, and used (20 g/tube, 250 U/g gel).

### Experimental Example 1.2. Test animals and feeding environment

Sixteen male beagle dogs (non-naive), which were over 3 years of age, weighed 9 to 13 kg, and had no oral clinical symptoms, were purchased from ORIENT BIO INC. and had a drug-free period of at least 1 month.

A feeding room with environmental conditions of 23 ± 3°C, 30 ± 10% relative humidity, ventilation frequency of 10 to 15 times/hour, 12 hours of light, and illuminance of 150 to 200 Lux was used. During the acclimatization and testing period, one animal per cage was raised individually wherein the cage was made of stainless steel. Feed and drinking water were provided ad libitum.

The reason why a beagle dog was chosen as a test animal was that the beagle dog is widely used in tests for oral-associated efficacy evaluation such as gingivitis, and it is easy to interpret and evaluate test results because abundant basic test data have been accumulated therefor.

### Experimental Example 1.3. Composition of test groups

Test groups G1 ("G1 Vehicle") and G2 ("G2 BASOD") were composed as follows.

| **Test group** | **Formulation** | **Administration route** | **Active ingredient** | **Dosage/dose frequency** | **Number of animals per group** |
|---|---|---|---|---|---|
| G1 | Oral gel containing no mixture of SOD and spores (vehicle control) | Oral application | - | 0.25 g/time two times /day | 8 |
| G2 | Oral gel containing mixture of SOD and spores | Oral application | Mixture of SOD and spores (2.5%) | 0.25 g/time two times/day | 8 |

### Experimental Example 1.4. Administration of test material

The test material of Experimental Example 1.1 was administered by being applied to the gums (gingival areas on the outside of both maxillary molars) every day for 4 weeks. The dosage was 0.25 g (62.5 U of SOD)/head per time twice a day.

### Experimental Example 1.5. Test evaluation items

### (1) Halitosis index

At week 0 (before starting administration), week 2, and week 4, halitosis was evaluated by a sensory test (see table below).

| Evaluation item | Evaluation method |
|---|---|
| Halitosis (Score: 1 to 5) | 1: No halitosis |
| | 2: Slightly perceptible level |
| | 3: Clearly perceptible level |
| | 4: Moderate halitosis |
| | 5: Severe foul-smelling halitosis |

### (2) Calculus index

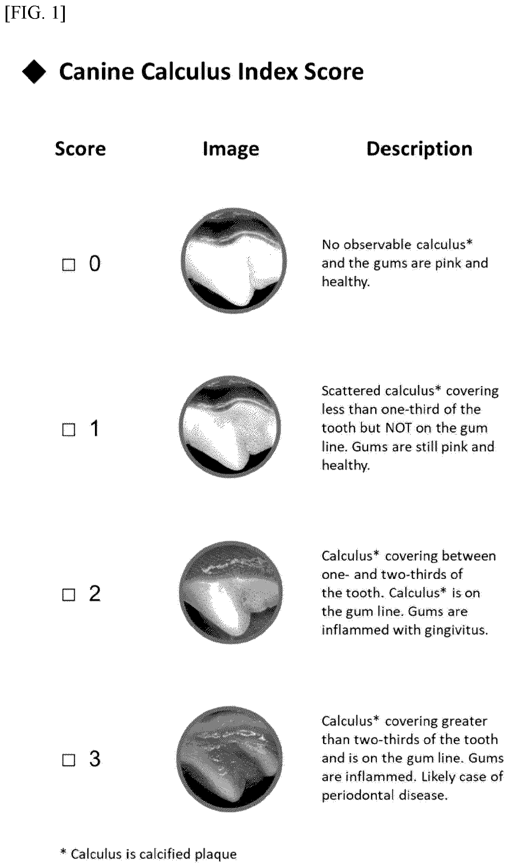

At week 0 (before starting administration), week 2, and week 4, the calculus index was calculated by opening the animal's mouth to expose the tooth surface, taking a photograph of the tooth surface, and performing naked-eye observation (see the table below and FIG. 1).

| Evaluation item | Evaluation method |
|---|---|
| Calculus (Score: 0 to 3) | 0: No calculus |
| | 1: Calculus covers less than one-third of the tooth surface, and gums are pink and healthy |
| | 2: Calculus covers about one-third to one-half of the tooth surface, and gingivitis is observed |
| | 3: Calculus covers greater than two-thirds of the tooth surface, and periodontitis is observed |

### (3) Plaque index

At week 0 (before starting administration), week 2, and week 4, the plaque index was calculated by staining plaque with a plaque colorant, taking a photograph of the tooth surface, and converting the area where the plaque colorant covered the tooth surface into a percentage of the total tooth surface (see table below).

| **Plaque Index** | | | **Thickness (colour intensity)** | |
|---|---|---|---|---|
| | **Coverage** | | | |
| | | | | |
| 0 = | no plaque | | | |
| 1 = | less than 25% of surface | | 1 = | light |
| 2 = | between 25-49% of surface | | 2 = | moderate |
| 3 = | between 50-74% of surface | | 3 = | heavy |
| 4 = | between 75% to 100% of surface | | | |

### (4) Gingival index

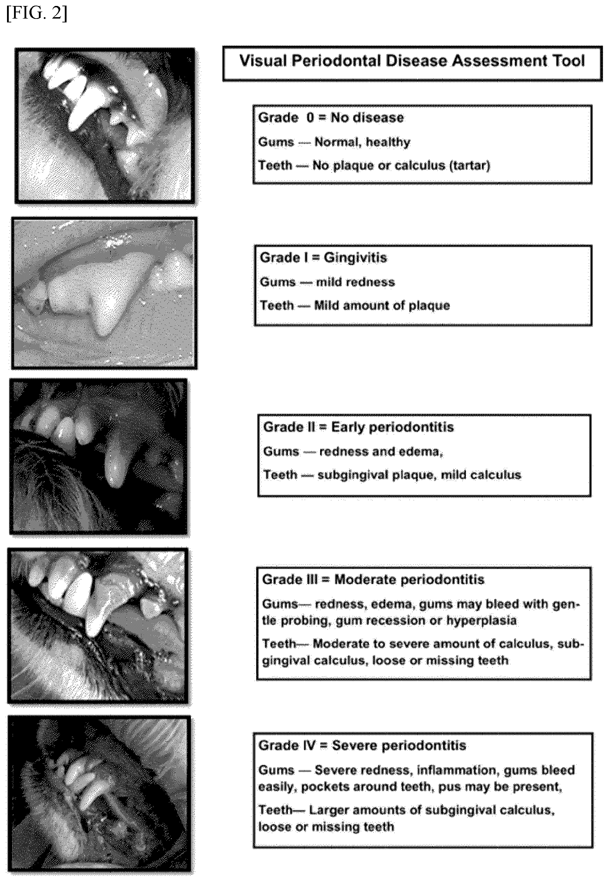

At week 0 (before starting administration), week 2, and week 4, the gingivitis index was calculated by opening the animal's mouth to expose the tooth surface and the gums around the teeth, taking a photograph thereof, and performing naked-eye observation (see the table below and FIG. 2).

| Evaluation item | Evaluation method |
|---|---|
| Gingivitis (Score: 0 to 4) | 0: No disease |
| | 1: Gingivitis |
| | Gums: mild redness, Teeth: mild amount of plaque |
| | 2: Early periodontitis |
| | Gums: redness and edema, Teeth: subgingival plaque, mild calculus |
| | 3: Moderate periodontitis |
| | Gums: redness, edema, gums may bleed, gum recession or hyperplasia |
| | Teeth: moderate to severe amount of calculus, subgingival calculus, loose or missing teeth |
| | 4: Severe periodontitis |
| | Gums: Severe redness, inflammation, bleeding, formation of pockets around teeth, pus may be present |

### (5) Other adverse reactions

In a case where other adverse reactions are observed during the administration of the test material, the symptoms were recorded.

### Experimental Example 1.6. Statistical method

To compare before and after treatment with the test material within the test group, statistical significance was tested using a paired sample t-test, and statistical processing was performed using the Graphpad prism 5.01 program that is a widely used commercial statistical package.

### Experimental Example 1.7. Test results and discussion

### (1) Halitosis index

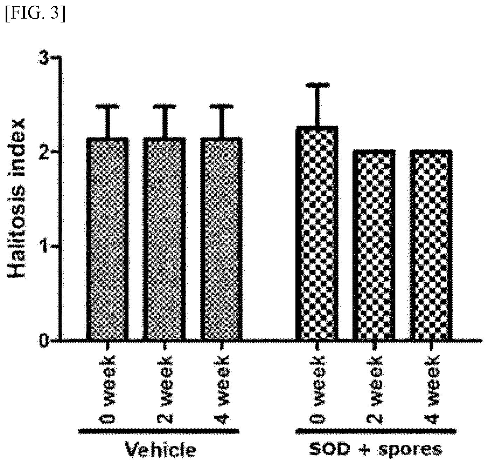

For all animals that were administered the mixture, no statistically significant observation could be made up to week 4 after administration, but it was found that the halitosis index tended to decrease (Table 3 and FIG. 3).

**[Table 3]**

| Group | Week | | |
|---|---|---|---|
| | 0 | 2 | 4 |
| G1_vehicle | 2.1 ± 0.35 | 2.1 ± 0.35 | 2.1 ± 0.35 |
| G2_BASOD | 2.3 ± 0.46 | 2.0 ± 0.00 | 2.0 ± 0.00 |

| | | | |
|---|---|---|---|
| Data represent the mean ± standard deviation (SD) | | | |

### (2) Calculus index

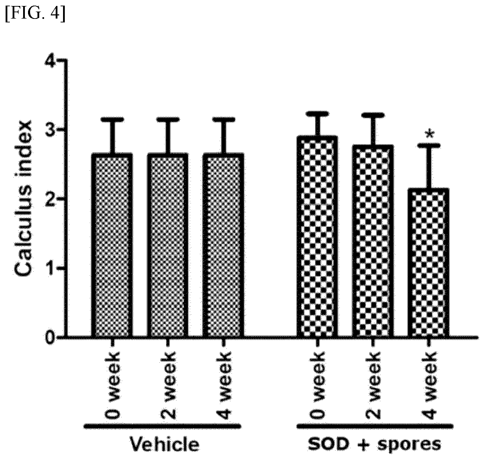

For all animals that were administered the mixture of SOD and spores, there was a tendency for calculus to be gradually removed up to week 4 after administration, and in particular, a statistically significant decrease in the calculus index was observed at week 4 as compared with week 0 (p <0.05) (Table 4 and FIG. 4). In Table 4 and FIG. 4, statistical significance was verified by a paired sample t-test.

**[Table 4]**

| | Week | | |
|---|---|---|---|
| Group | 4 | 2 | 4 |
| G1_vehicle | 2.6 ± 0.52 | 2.6 ± 0.52 | 2.6 ± 0.52 |
| G2_BASOD | 2.9 ± 0.35 | 2.8 ± 0.46 | 2.1 ± 0.64* |

| | | | |
|---|---|---|---|
| Data represent the mean ± standard deviation (SD). | | | |

### (3) Plaque index

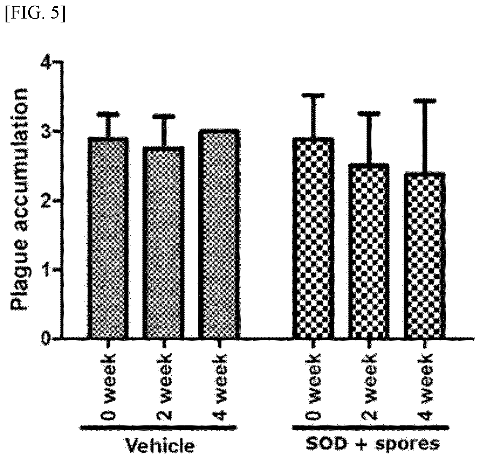

For all animals that were administered the mixture of SOD and spores, no statistically significant observation was made up to week 4 after administration, but it was found that the plaque index tended to decrease (Table 5 and FIG. 5).

**[Table 5]**

| | Week | | |
|---|---|---|---|
| Group | 0 | 2 | 4 |
| G1_vehicle | 2.9 ± 0.35 | 2.8 ± 0.46 | 3.0 ± 0.00 |
| G2_BASOD | 2.9 ± 0.64 | 2.5 ± 0.76 | 2.4 ± 1.06 |

| | | | |
|---|---|---|---|
| Data represent the mean ± standard deviation (SD) | | | |

### (4) Gingivitis index

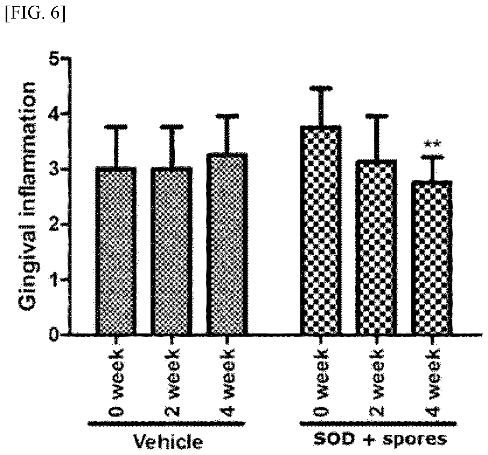

For all animals that were administered the mixture of SOD and spores, the efficacy of eliminating gum bleeding and ameliorating edema and redness was observed up to week 4 after administration, and in particular, a statistically significant decrease in the gingivitis index was observed at week 4 as compared with week 0 (p<0.01) (Table 6 and FIG. 6). In Table 6 and FIG. 6, statistical significance was verified by a paired sample t-test.

**[Table 6]**

| | Week | | |
|---|---|---|---|
| Group | 0 | 2 | 4 |
| G1_vehicle | 3.0 ± 0.76 | 3.0 ± 0.76 | 3.3 ± 0.71 |
| G2_BASOD | 3.8± 0.71 | 3.1 ± 0.83 | 2.8 ± 0.46** |

| | | | |
|---|---|---|---|
| Data represent the mean ± standard deviation (SD) | | | |

### (5) Discussion

The test material, which is a mixture of SOD and spores, was applied to the gums of the beagle dogs twice daily at a dose of 0.25 g (62.5 U of SOD)/head. As a result, it was found that the halitosis index, calculus index, plaque index, and gingivitis index all tended to decrease from week 2 to week 4 after administration as compared with week 0. In particular, for calculus and gingivitis, statistically significant amelioration of symptoms was observed as compared with week 0. In addition, no special adverse reactions occurred during the entire test process, from which it was identified that the test material was not harmful.

Based on the above results, it was found that the test material, which is a mixture of SOD and spores, is useful for ameliorating an oral disease such as halitosis, calculus, plaque, and gingivitis.

### Experimental Example 2. Animal tests: cats

Tests using pet cats were performed to identify whether the superoxide dismutase (SOD) purified in Example 1 is effective in promoting oral health and managing oral hygiene.

### Experimental Example 2.1. Test material

The test material used was prepared in the same manner as in Experimental Example 1.1, except for the *Bacillus amyloliquefaciens* GF423 strain spores.

### Experimental Example 2.2. Test animals

Regardless of gender or breed, among the pet cats that were over 6 months old and weighed between 1 kg and 10 kg, diseased cats showing oral symptoms such as halitosis, decreased energy due to pain, difficulty chewing, decreased appetite, drooling, and gum redness (caused by erosion and proliferative ulcer) were randomly selected as test cats.

### Experimental Example 2.3. Composition of test groups

Consent to voluntarily participate in the clinical trial was obtained from the guardian of each test cat. Then, the test cats were randomly allocated.

| **Symbol** | **Test group** | **Administration route** | **Dosage** | **Number of animals** |
|---|---|---|---|---|
| G1 | Vehicle | Oral application | 0.3 g/time | 9 |
| | | | two times /day | |
| G2 | SOD-administered group | Oral application | 0.3 g/time | 8 |
| | | | two times/day | |

For test group G1, the breed was mixed breed, Korean short hair (KSH), Exotic, or Siamese, the sex was neutered male (NM) or spayed female (SF), and the age was 2 to 10 years.

For test group G2, the breed was Turkish Angora (T.A.) or Korean short hair (KSH), the sex was neutered male (NM) or spayed female (SF), and the age was 1 to 11 years old.

### Experimental Example 2.4. Administration of test material

The test material of Experimental Example 2.1 was administered by being applied to the gingival area of the right maxillary molar every day for 4 weeks. The dosage was 0.3 g (75 U of SOD)/head per time twice a day.

### Experimental Example 2.5. Test evaluation items

Before the start of the clinical trial, blood tests (alanine aminotransferase (ALT) and the like) and basic physical examinations (body weight and the like) were conducted for the test cats.

### (1) Feed intake

The feed intake was evaluated for each test cat at week 0 (before starting administration), week 2, and week 4 (see table below).

| Evaluation item | Evaluation method |
|---|---|
| Feed intake (Score: 1 to 4) | 1: Normal or no change |
| | 2: Feed intake decreased by up to 1/3 |
| | 3: Feed intake decreased by 1/3 to 2/3 |
| | 4: Feed intake decreased by more than 2/3 |

### (2) Activity (mood)

The activity (mood) was observed and evaluated for each test cat at week 0 (before starting administration), week 2, and week 4 (see table below).

| Evaluation item | Evaluation method |
|---|---|
| Activity (mood) (Score: 1 to 4) | 1: Normal or no change |
| | 2: A little down |
| | 3: Moderately down |
| | 4: Severely down |

### (3) Palpable pain index

Palpable pain was evaluated for each test cat at week 0 (before starting administration), week 2, and week 4 by observing the reaction that occurred in a case of touching the diseased area of the test cat with the hand (see table below).

| Evaluation item | Evaluation method |
|---|---|
| Palpable pain (Score: 1 to 4) | 1: Normal |
| | 2: Slight pain (slight nociceptive response to strong palpation) |
| | 3: Moderate pain (moderate nociceptive response to moderate palpation) |
| | 4: Severe pain (severe nociceptive response to mild palpation) |

### (4) Halitosis index

Halitosis was evaluated by a sensory test at week 0 (before starting administration), week 2, and week 4 (see table below).

| Evaluation item | Evaluation method |
|---|---|
| Halitosis (Score: 1 to 5) | 1: No halitosis |
| | 2: Slightly perceptible level |
| | 3: Clearly perceptible level |
| | 4: Severe foul-smelling halitosis |

### (5) Plaque index

At week 0 (before starting administration), week 2, and week 4, the plaque index was calculated by staining the right molar with a plaque colorant and converting the area where the plaque colorant covered the tooth surface into a percentage of the total tooth surface (see table below), and a photograph thereof was taken.

| **Plaque Index** | | | **Thickness (colour Intensity)** | |
|---|---|---|---|---|
| | **Coverage** | | | |
| | | | | |
| 0 = | no plaque | | | |
| 1 = | less than 25% of surface | | 1 = | light |
| 2 = | between 25-49% of surface | | 2 = | moderate |
| 3 = | between 50-74% of surface | | 3 = | heavy |
| 4 = | between 75% to 1 00% of surface | | | |

### (6) Calculus index

At week 0 (before starting administration), week 2, and week 4, the calculus index was calculated by performing naked-eye observation of calculus on the right maxillary molar (see table below and FIG. 1), and a photograph thereof was taken.

| Evaluation item | Evaluation method |
|---|---|
| Calculus (Score: 0 to 3) | 0: No calculus |
| | 1: Calculus covers less than one-third of the tooth surface, and gums are pink and healthy |
| | 2: Calculus covers about one-third to one-half of the tooth surface, and gingivitis is observed |
| | 3: Calculus covers greater than two-thirds of the tooth surface, and periodontitis is observed |

### (7) Gingivitis index

At week 0 (before starting administration), week 2, and week 4, the gingivitis index was calculated by performing naked-eye observation of the gingival area of the right maxillary molar, measuring a score based on the table below, and then calculating a total score, and a photograph thereof was taken.

| Evaluation item | Redness | Edema | Bleeding |
|---|---|---|---|
| 0 points | No disease | No disease | No disease |
| 1 point | Mild redness | Mild edema | Bleeding when pressed |
| 2 points | Moderate redness | Moderate edema | Spontaneous bleeding |
| 3 points | Severe redness | Severe edema | Ulcer |

### (8) Other adverse reactions

All adverse reactions (loss of appetite, vomiting, diarrhea, hematochezia, hematemesis, lethargy, gastrointestinal disorder, and the like), which occurred during the period of administration of the test material to identify its effectiveness were observed and recorded by the guardian or test administrator.

### Experimental Example 2.6. Statistical method

To compare before and after treatment with the test material within the test group, statistical significance was tested using a paired sample t-test, and statistical processing was performed using the Graphpad prism 5.01 program that is a widely used commercial statistical package.

### Experimental Example 2.7. Test results and discussion

### (1) Feed intake and body weight

No significant differences in feed intake and body weight were observed in any group during the test period (data not shown).

### (2) Activity

For the vehicle control group, significant improvement in activity was observed starting from week 2; and for the SOD group, activity tended to gradually increase, but there was no statistical significance (data not shown).

### (3) Palpable pain index

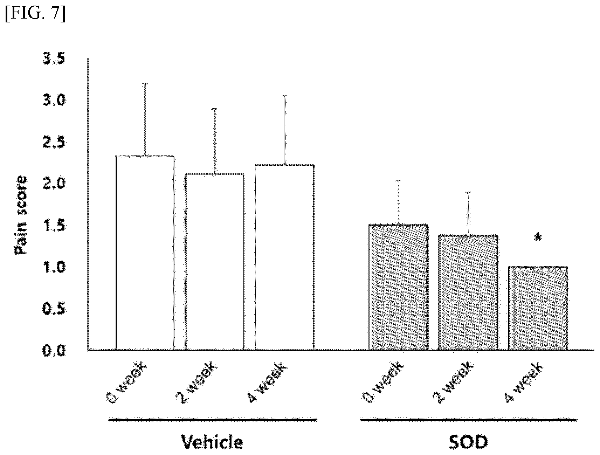

For the vehicle control group, there was little change in palpable pain until the end of the test; however, for the SOD group, palpable pain decreased starting from week 2 and statistically significant amelioration in palpable pain was observed at week 4 (FIG. 7).

### (4) Halitosis index

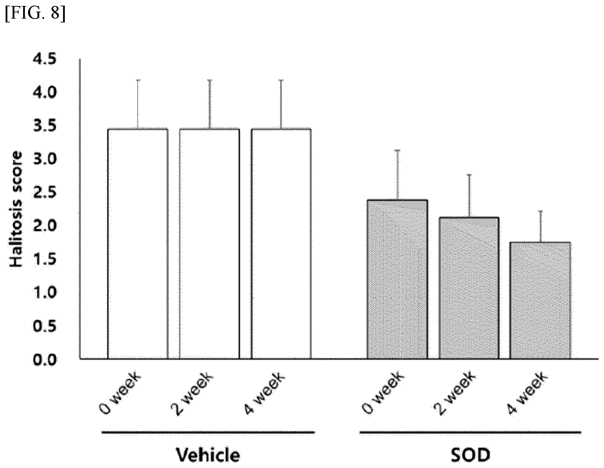

For the vehicle control group, the halitosis index did not change until the end of the test; however, for the SOD group, it was found that the halitosis index tended to continuously decrease (FIG. 8).

### (5) Plaque index

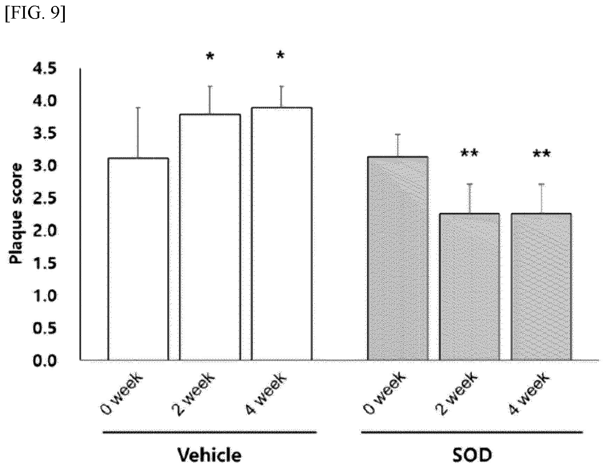

For the vehicle control group, the plaque index clearly increased during the test period; however, for the SOD group, the plaque index decreased with statistical significance starting from week 2 until the end of the test (FIG. 9).

### (6) Calculus index

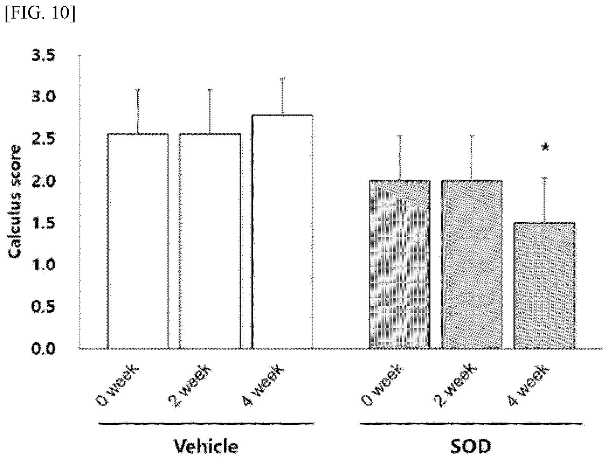

For the vehicle control group, the calculus index gradually increased; however, for the SOD group, the calculus index decreased with statistical significance at week 4 (FIG. 10).

### (7) Gingivitis index

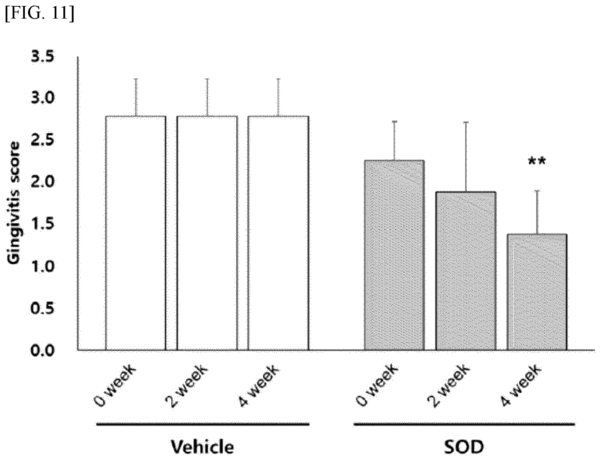

For the vehicle control group, there was no significant change in the gingivitis index; however, for the SOD group, the gingivitis index decreased starting from week 2 and statistically significant decrease in the gingivitis index was observed at week 4 (FIG. 11).

### (8) Discussion

The test material SOD was applied to the gums of the pet cats twice daily at a dose of 0.3 g (62.5 U of SOD)/head per time. As a result, it was found that the halitosis index, palpable pain index, plaque index, calculus index, and gingivitis index all tended to decrease from week 2 to week 4 after administration as compared with week 0. In particular, for palpable pain, plaque, calculus, and gingivitis, statistically significant amelioration of symptoms was observed as compared with week 0. In addition, no special adverse reactions occurred during the entire test process, from which it was identified that the test material was not harmful.

Based on the above results, it was found that even in a case of being administered alone, the test material SOD is not only useful for ameliorating an oral disease such as halitosis, calculus, plaque, and gingivitis, but is also useful for decreasing pain caused by such an oral disease.

## Claims

1. An oral composition, comprising as an active ingredient at least one selected from the group consisting of a superoxide dismutase (SOD) and a *Bacillus sp.* strain spore.

2. The oral composition of claim 1, wherein the composition comprises the SOD and the *Bacillus sp.* strain spore.

3. The oral composition of claim 1, wherein the SOD is derived from a *Bacillus sp*. strain.

4. The oral composition of claim 3, wherein the *Bacillus sp.* strain comprises *Bacillus amyloliquefaciens* GF423 strain (KCTC 13222 BP) or *Bacillus amyloliquefaciens* GF424 strain (KCTC 13227 BP).

5. The oral composition of claim 3, wherein the SOD comprises Mn-SOD or deamidated Mn-SOD.

6. The oral composition of claim 1, wherein the SOD comprises the amino acid sequence of SEQ ID NO: 2 or SEQ ID NO: 4.

7. The oral composition of claim 1, wherein the *Bacillus sp.* strain spore comprises a *Bacillus amyloliquefaciens* GF423 strain (KCTC 13222 BP) spore or a *Bacillus amyloliquefaciens* strain GF424 (KCTC 13227 BP) spore.

8. The oral composition of claim 1, wherein the composition is used for promoting oral health or managing oral hygiene.

9. The oral composition of claim 1, wherein the composition is used for preventing or improving oral pain, halitosis, calculus, plaque, dental caries, or a periodontal disease.

10. The oral composition of claim 1, wherein the composition is in a form of a gel or paste.

11. An oral product, comprising the composition of any one of claims 1 to 10.

12. The oral product of claim 11, wherein the product comprises any one selected from toothpaste, mouthwash, oral gargle, gum, candy, oral spray, oral gel, oral ointment, oral patch, and oral rinse.

13. A pharmaceutical composition for prevention or treatment of an oral disease, comprising the composition of any one of claims 1 to 10.

14. A veterinary composition, comprising the composition of any one of claims 1 to 10.

15. A food composition, comprising the composition of any one of claims 1 to 10.

16. A feed composition, comprising the composition of any one of claims 1 to 10.

17. A method for preventing or treating an oral disease, comprising administering to an individual at least one selected from the group consisting of a superoxide dismutase (SOD) and a *Bacillus sp.* strain spore.

18. A method of promoting oral hygiene, comprising administering to an individual at least one selected from the group consisting of a superoxide dismutase (SOD) and a *Bacillus sp.* strain spore.

19. The method of claim 17 or 18, wherein the method comprises administering to the individual the SOD and the *Bacillus sp.* strain spore.

20. The method of claim 19, wherein the SOD and the *Bacillus sp.* strain spore are administered as a single composition or as separate compositions comprising each of them.

21. The method of claim 19, wherein the SOD and the *Bacillus sp.* strain spore are administered simultaneously, sequentially, or in reverse order.
